# EUROPEAN PATENT SPECIFICATION

(11) **EP 1 534 237 B1**
(45) Date of publication and mention of the grant of the patent: **04.02.2009**
(21) Application number: 03757165.0
(22) Date of filing: 04.06.2003
(51) Int. Cl.: A61K 9/00, A61K 47/26, A61K 47/38, A61K 47/36

(54) **ORALLY DISINTEGRATING TABLETS AND PROCESS FOR OBTAINING THEM.**
IM MUND ZERFALLENDE TABLETTEN UND VERFAHREN ZU IHRER HERSTELLUNG.
COMPRIMÉS À DÉSINTEGRATION ORALE ET LEUR PROCÉDÉ DE FABRICATION

(30) Priority: 10.06.2002 ES 200201440
(43) Date of publication of application: 01.06.2005
(73) Proprietor: VITA CIENTIFICA, S.L., 08970 Sant Joan Despi (ES)
(72) Inventor: SEGADO FERRAN, Javier, E-08026 Barcelona (ES)
(74) Representative: Ponti Sales, Adelaida
(86) International application number: PCT/IB2003/002446
(87) International publication number: WO 2003/103629

(56) References cited:
- WO-A-01/12161
- US-A- 5 686 107
- US-A- 5 904 937
- NILSSON P ET AL: "PHYSICOCHEMICAL ASPECTS OF DRUG RELEASE V. THE IMPORTANCE OF SURFACE COVERAGE AND COMPACTION ON DRUG DISSOLUTION FROM ORDERED MIXTURES" INTERNATIONAL JOURNAL OF PHARMACEUTICS (AMSTERDAM), vol. 45, no. 1-2, 1988, pages 111-122, XP009018006 ISSN: 0378-5173
- MATTSSON S ET AL: "Formulation of high tensile strength rapidly disintegrating tablets: Evaluation of the effect of some binder properties" S.T.P. PHARMA SCIENCES 2001 FRANCE, vol. 11, no. 3, 2001, pages 211-220, XP009018012 ISSN: 1157-1489

## Description

### Field of the invention

This invention relates to orally disintegrating tablets, in other words, tablets for peroral administration which disintegrate quickly in the cavity of the mouth, in particular in less than 30 seconds, and to the process for obtaining them.

### Background of the invention

The development of solid formulas that disintegrate quickly in the mouth without requiring water has awoken great interest in the advantages this implies for patients who have difficulty in swallowing, such as old people, infants, patients with mental problems and non-cooperative patients, as well as the population in general, since it makes it possible for the drug to be administered without the need for water.

In the European Pharmacopoeia 4th edition, Supplement 4.1, published in October 2001, orally disintegrating tablets are defined as non-coated tablets for placing in the mouth which disintegrate quickly before they are swallowed. It also establishes 3 minutes as the time under which they must disintegrate in the disintegration test for tablets and capsules, according to the Ph. Eur. 2.9.1. method.

Different technologies have been developed, based on alternatives to the conventional processes used for obtaining tablets, which enable the obtaining of formulas that disintegrate quickly in the oral cavity, and which are very palatable. The most well-known include those which make it possible to obtain oral lyophilisate, matrixes by compression of saccharide based shearform floss particles and films or wafers. However, the compositions obtained using said technologies have disadvantages to a greater or lesser extent, such as their being highly fragile, extremely sensitive to atmospheric humidity, technologically difficult to obtain and especially costly to produce on an industrial scale.

To simplify the aforementioned technologies and in particular to reduce production costs and overcome the aforementioned disadvantages, the standard tablet production processes have been optimised.

The most frequently used processes for obtaining tablets include:
a) Obtaining tablets by the direct compression of mixtures that contain at least one inorganic excipient that is insoluble in water, for example, calcium phosphate, one or more disintegrants, for example, crospovidone and optionally, water soluble excipients. Said technology is registered as Ziplets^{®} by Eurand and is described in the international application patent WO 9944580. However, the compositions used contain a high percentage of insoluble excipients which leave a high amount of residue in the mouth and jeopardise their palatability.
b) Obtaining tablets via the direct compression of mixtures that contain at least a non-direct compression filler, for example, dextrose, mannitol, sorbitol, lactose, and a lubricant. Said technology is registered as Durasolv^{®} by Cima, and is described in the patent US 6.024.981.
c) Obtaining multiparticulate tablets made up of mixtures of microencapsulated active ingredients and excipients that contain one or several disintegrating agents, one or several hygroscopic agents and a direct compression soluble diluent. Said technology is registered as Flashtab^{®} by Prographarm and is described in the patent EP 0548356.
d) Obtaining orally disintegrating tablets that disintegrate in the oral cavity in less than 60 seconds, and which contain spray-dried mannitol, crospovidone and other excipients, by direct compression. Said technology is described in the patent application WO 00/57857 by Yuhan Corporation.

However, all the above processes for obtaining tablets involve, to a greater or lesser extent, the following disadvantages:
- A high content of insoluble excipients or microencapsulated active ingredients that give the formula a gritty feel after they have been disintegrated in the oral cavity and, consequently, problems with palatability.
- Excessively long disintegration times in comparison with oral lyophilisates or wafers, which, in general, dissolve in less than 10 seconds.
- Insufficient mechanical resistance to resist conventional packaging and transport operations.

### Description of the invention

A first aspect of the present invention is to provide tablets for oral administration that disintegrate quickly in the oral cavity, in particular, in less than 30 seconds, and which can hardly be noticed on the tongue after their disintegration.

A second aspect of the present invention is to.provide a process for obtaining said orally disintegrating tablets via direct compression, where direct compression is understood as a manufacturing process that involves sieving, mixing and compression operations only.

### Detailed description of the invention

Surprisingly, the present invention has revealed that by using a diluent of high dissolution rate and high compressibility, and limiting the proportion and size of the particle of the insoluble ingredients, mixtures with optimum compressibility can be obtained. These mixtures enable the obtaining of orally disintegrating tablets which disintegrate in the mouth in less than 30 seconds, preferably less than 20 seconds, once they come into contact with saliva in the oral cavity, and which are hardly noticed on the tongue.

A further advantage is that the tablets described in the invention have sufficient mechanical resistance to resist the production and distribution operations, unlike other fast disintegration formulas such as oral lyophilisates, tablets of saccharide based shearform floss and wafers. The tablets of the invention have a friability of below 0.5%, preferably below 0.20, as specified by Ph. Eur. 2.9.7. These friability values enable packaging in any kind of package using conventional machinery, and do not require any special care to be taken in the intermediate bulk storage of the tablets or in the feed systems used in the packaging operation.

As a result, the first aspect of the present invention relates to an tablet for oral administration as defined in the attached claims 1 to 11.
A priori, there are no limitations to the active ingredients in this invention, although the active ingredients indicated in patients with swallowing difficulties, such as infants or old patients and/or non-cooperative patients, for example, patients with mental problems, are preferential candidates.

Of special interest are the active ingredients with dosage preferably below 50 mg per tablet. The preferred compounds are selected from, but not limited to, the following: anti-ulcer drugs: famotidine; antiemetics: ondansetron, granisetron, dolasetron, domperidone, metoclopramide; antihypertensive drugs: analapril, losartan, candesartan, valsartan, lisinopril, ramipril, doxazosin, terazosin; antihistaminic drugs: loratadine, cetirizine; antipsychotic drugs: risperidone, olanzapine, quetiapine; antidepressants: paroxetine, fluoxetine, mirtazapine; analgesics and anti-inflammatory drugs: piroxicam; antihypercholesterolemic drugs: simvastatin, lovastatin, pravastatin; antimigraine drugs: zolmitriptan, naratriptan, rizatriptan; anti-epileptic drugs: lamotrigine; anti-Parkinson drugs: selegiline, apomorphine; anxiolytic drugs: diazepam, lorazepam, zolpidem; anti-asthma dugs: zafirlukast, montelukast; erection dysfunction agents: sildenafil; both in their free base form and in their acceptable pharmaceutical salts, hydrates, solvates or isomers.

The orally disintegrating tablets described in the present invention disintegrate in less than 30 seconds, preferably in less than 20 seconds, once they come into contact with the saliva of the oral cavity. To determine the disintegration time, an alternative *in vitro* method has been standardised which is more discriminating than that which is set forth in Ph. Eur. 2.9.1., together with an *in vivo* disintegration test. The values obtained in both tests have been seen to be reproducible and are related, where the *in vivo* results are always lower than those obtained *in vitro* (see Experimental Section, Example 1). The tests used are described below in the "tablet characterisation" section set forth in the Experimental Section of this invention.

Spray-dried mannitol, an excipient which is commercially available, such as Mannogen^{™} EZ *spray dried mannitol* by SPI Pharma and Pearlitol^{®} SD by Roquette, has physical-chemical properties that make it ideal for constituting the appropriate diluent for this invention. The following is of particular interest:
- It dissolves easily in water (1 in 5.5 parts at 20°C);
- It dissolves quickly in water (5 g dissolve in approximately 5 s in 150 mL of water at 20°C). This disintegrating rate is much faster than that of direct compression mannitol, that of powder mannitol and other related saccharide excipients. Spray-dried mannitol is made up fundamentally by the crystalline form α, unlike the other types of mannitol, which are made up of the β form. Both forms can be easily distinguished using the IR spectrum.
- It has optimum fluidity for direct compression processes (flowability: 6 seconds and ability to settle: 16-18 ml).
- It is highly compressible (Cohesion Index: 1500 - 2000).
- It has good dilution capacity due to the size and form of the particle, which makes it possible to accept large amounts of active ingredients that are not easily compressed.
- This is a product with a deformation by fragmentation when it is subjected to pressure, generating new particle surfaces and becoming insensitive to the loss of compressibility due to over lubrication with hydrophobic lubricants.
   It is very chemically stable; non-hygroscopic and does not form Maillard reactions with amino groups like other related saccharide excipients.
- It has optimum organoleptic properties due to negative dissolution heat (sense of freshness), its sweetening power of approximately 50% of that of sucrose, and its excellent palatability due to its small particle size.

It has been established that the compounds of the present invention must contain at least 59.5% of spray-dried mannitol.

With regard to the dissolving capacity of spray-dried mannitol, in general, it has been established that to guarantee the compressibility and fluidity of the mixture that is to be compressed, the active ingredient content must not exceed 10% in weight of the total weight of the tablet. Also, to guarantee the palatability of the finished product and the uniformity of the mixture, the active ingredient must be a fine powder, where at least 90% in weight of the active ingredient has a particle size of below 100 µm.

To minimise the disintegration time and maximise the mechanical resistance of the tablets of this invention, a disintegration promoter system has been designed, made up of the following:
- Microcrystalline cellulose (e.g. Avicel® PH 101 or Emcocel® 50 M) of average particle size of approximately 50 µm, where at least 99% in weight of microcrystalline cellulose is below 250 µm. The proportion of microcrystalline cellulose is from 10 to 18% in weight of the total weight of the tablet, preferably from 12 to 15%. Said amount makes it possible to significantly improve compressibility, reduce friability and achieve a substantial reduction in disintegration time. Higher quantities have a negative impact on the palatability of the formula and lower quantities worsen the capacity of the disintegration promoter.
   Sodium croscarmellose (e.g. Ac-Di-Sol^{®}) is present in a proportion from 1 to 4% of the total weight of the tablet, preferably from 2 to 3%. Higher quantities have a negative impact on the palatability of the formula and do not offer significant advantages with regard to disintegration rate.
- Optionally, a humidity absorbent agent may be added, such as precipitated silica (e.g. Syloid^{®}) in a proportion from 0.1 to 0.5% in weight of the total weight of the tablet, which may counteract the hydrophobicity of certain active ingredients and improve the fluidity of the mixture.

Preferably, said disintegration promoter system should be in a proportion from 14 to 18.5% of the total weight of the mixture.

The tablets of this invention may also contain, to improve patient acceptance, a sweetening/flavouring system made up of:
- An artificial sweetener or a combination thereof which must be adapted in accord with the organoleptic properties of the active ingredient. The following may be used, but the list does not exclude other options: aspartame, sodium cyclamate, sodium saccharine, ammonium glycyrrhizinate, neohesperidine dihydrochalcone. The artificial sweetener content is from 0.5 to 2% in weight of the total weight of the tablet.
- A flavouring agent, preferably a microencapsulated powder flavouring on a support that is soluble and which disintegrates in water. The flavouring content is from 0.5 to 2% in weight of the total weight of the tablet.

Optionally, ionic exchange resins or polymers which form complexes with the active ingredients may be added, enabling masking of unpleasant tastes. The following may be used, but the list does not exclude other options: polividone, β-ciclodextrin, potassium polacrilin.

Especially good results regarding the masking of unpleasant tasting active ingredients have been obtained using the system made up of aspartame, ammonium glycyrrhizinate, mentholated flavouring and L-menthol (0.1-0,2% in weight), which due to its refreshing effect has a synergic effect with the spray-dried mannitol and a good tastemasking capacity due to its residual effect. Therefore, the composition of the invention with this sweetening/flavouring system is beneficial in that it avoids the use of costly processes such as microencapsulation or coating the active ingredients in order to mask their bitter taste.

Finally, to facilitate the compression operation, a lubricant agent must be added and, if necessary, an anti-adherent agent in an appropriate proportion. Although the preferred lubricant is magnesium stearate, other less hydrophobic lubricants may be used to counter the hydrophobicity in certain cases of specific active ingredients such as sodium fumarate, polyethylene glycol 6000, sodium lauryl sulphate and a combination of magnesium stearate with sodium lauryl sulphate (9:1) and sucrose esters. The proportion of lubricant shall be from 0.5 to 2% in weight of the total weight of the tablet. The proportion of anti-adherent agent, such as talcum, colloidal silicon dioxide, shall be from 0.5 to 2% in weight of the total weight of the tablet.

Another advantage is that palatability improves even more if the proportion of insoluble ingredients is below 20%. Insoluble ingredients of the composition of the invention include: microcrystalline cellulose, sodium croscarmellose, humidity adsorbing agent, lubricant agents, anti-adherent agents and insoluble active ingredients.

The present invention shows that it is possible to have a significant influence on the disintegration rate of the tablet by modifying the dimensions and shape of the tablet. In general, the thinner the tablet and the greater its porosity, the sooner the structure of the matrix is weakened when it comes into contact with saliva, since the disintegration process is produced after wetting all the die via capillary action. Also, any shape which maximises the contact surface with the saliva will produce a significant reduction in disintegration time, obtaining disintegration values of up to below 20 seconds. The preferred shape of this invention is a flat round bevelled tablet with a thickness from 2.2 to 1.8 mm, though this is not exclusive.

Thus, the mixtures of the aforementioned components shall be transformed into orally disintegrating tablets in accord with the process for obtaining them described below and defined in the attached claims 12 to 14. According to the invention, the tablets have:
- A friability below 0.5%, preferably below 0.2 %.
- A disintegration time in the oral cavity of below 30 seconds, preferably below 20 seconds.
- An apparent density from 1.1 to 1.3 g/ml.

The apparent density of the tablets is calculated by means of the division of the mass (m) by the volume (.e.g. V=π·r²·h, if the tablet is flat and round like the preferable shape proposed in this invention, where r is the radius and h the thickness of the tablet). It has been shown that the apparent densities of the tablets obtained with the compositions of the present invention correlate to the resistance to breakage of the tablets and to their disintegration time in the mouth. It has also been shown that tablets with apparent densities from 1.1 to 1.3 g/ml make it possible to guarantee the specifications of friability and disintegration, which is the aim of the present invention.

It has also been observed that in order to guarantee fulfilment of the specification of the disintegration time in the oral cavity, the tablets should disintegrate in less than 40 seconds in the *in vitro* disintegration test described in the tablet characterisation section of the Experimental Section of the present invention.

As mentioned previously, the present invention also relates to a process for obtaining said orally disintegrating tablets comprising direct compression. The tablets described in the invention are obtained by compression of a powder blend into solid form, which dimensions and shape enable even further minimisation of disintegration time.

In particular, the process for obtaining an tablet for oral administration as previously defined comprises the following steps:
i) Sieving and mixing of the components except for the lubricant agent;
ii) Sieving of the lubricant agent;
iii) Mixing all the components; and
iv) Direct compression of the final mixture.

In some cases, sequential mixing processes may be required in order to guarantee the uniformity of the content of the mixture or to guarantee the functionality of certain excipients (e.g. mixtures of active ingredient with polymers for taste masking).

Due to the high compressibility of the compositions of the present invention, it is possible to obtain tablets with appropriate mechanical resistance, applying low pressures during the compression process, preferably from 3 to 10 kN.

Mixtures which are considered appropriate for compression are the ones which possess a flowability below or equal to 10 seconds, determined according to the method described in Ph. Eur. 2.9.16 and/or an ability to settle (V₁₀-V₅₀₀) below or equal to 20 ml, determined in accord with Ph. Eur. 2.9.15.

Preferably, the mixture must also possess a preferential cohesion index (CI) of over 700, being CI the slope of the straight line that adjusts the hardness values (Newtons) in accord with the strength of compression (decaNewtons), multiplied by 10⁵.

### Description of the figures

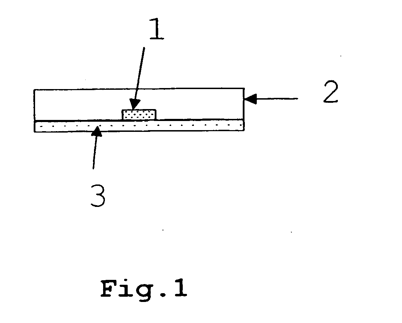

Figure 1 shows schematically the *in vitro* disintegration test. In said figure 1, tablet 1 is placed in a Petri dish 2 on a filter paper with 9-10 ml of disintegration medium 3.

### Experimental Section

Particular embodiments are shown by the following examples without limiting the scope of the invention.

### General process:

- Weigh all components of the formula.
- Sieve, except for the lubricant, through a 0.5 mm sieve.
- Mix in a Túrbula T2B mixer for 5 minutes.
- Sift the lubricant through a 0.32 mm sieve.
- Mix in a Túrbula T2B mixer for 2 minutes.
- Compress in a machine fitted with the appropriate compression tools, in accord with specifications of established weight, thickness and hardness.

### Characterisation of tablets:

### Hardness (N):

This is determined in a Schleuniger 6D durometer using the resistance to crushing method set forth in en Ph. Eur. 2.9.8. The average value and range of the determinations are detailed.

### Weight (mg) :

This is determined by an analytical weighing balance with a sample of 10 tablets. The average value and range of the determinations are detailed.

### Thickness (mm) :

This is determined with a calliper square using a sample of 10 tablets. The average value and range of the determinations are detailed.

### Friability (%):

This is determined in a Pharmatest friability tester using the method set forth in Ph. Eur. 2.9.7.

### Tensile strength (N/mn²):

This is calculated based on the average values of hardness and thickness in accord with the formula T = 2·F/π·d·h;
where "F" is resistance to crushing, "d" is the diameter of the tablet and "h" is the thickness.

### In vitro disintegration test (s):

On a 100x10 mm glass Petri dish, place a 90 mm diameter filter paper (reference: WH 1442090) and pour on said dish a volume of 9-10 ml of disintegration medium at room temperature (aqueous solution at 10% (w/w) of cobalt II 6-hydrate chloride). Tilt the dish until all the paper is soaked and there are no air bubbles below it. Immediately after the preparation, place a tablet on the dish and start the chronometer. Observe how the water rises by capillary action and the final point of disintegration is taken to be when the tablet is fully wet. Six tablets are tested on each dish (see figure 1: *in vitro* disintegration test):

### In vivo disintegration test (s):

Place the orally disintegrating tablet on the tongue, start the chronometer and actively suck until it is completely disintegrated. Total disintegration is considered to have been reached when the tablet has completely broken down in the mouth, even though there may still be residue to be swallowed. Note down the time in seconds. Perform the test with a maximum of three tablets.

### EXAMPLE 1

A placebo of orally disintegrating tablets was obtained using the general process described initially and the composition given in Table I. Table I gives a summary of the results obtained in the characterisation of the tablets. Tables II and III compile the results obtained in the *in vitro* and *in vivo* disintegration tests by two different analysts.

**Table I: Orally disintegrating placebo tablets**

| **Composition for 1000 tablets** | |
|---|---|
| **Ingredients** | **quantity (g)** |
| Spray-dried mannitol | 108.0 |
| Microcrystalline cellulose | 22.5 |
| Sodium croscarmellose | 4.5 |
| Aspartame | 2.0 |
| Mint flavouring | 2.0 |
| Magnesium stearate | 3.0 |
| **Parameters** | **Values** |
| Shape | round 9.2 mm, flat, bevelled |
| Average weight (mg) | 141.8 (135.2-146.9) |
| Hardness (N) | 21 (15 - 28) |
| Thickness (mm) | 1.94 (1.85 - 1.99) |
| Tensile strength (N/mm²) | 0.7 |
| Friability (%) | 0.35 |
| *in vitro* disintegration time (s) | See Table II |
| *in vivo* disintegration time (s) | See Table III |

**Table II: In vitro disintegration time (seconds)**

| **Orally disintegrating placebo tablets Example 1** | | |
|---|---|---|
| **Num.** | **ANALYST 1** | **ANALYST 2** |
| **1** | 26 | 27 |
| **2** | 32 | 28 |
| **3** | 19 | 23 |
| **4** | 14 | 13 |
| **5** | 12 | 25 |
| **6** | 17 | 30 |
| **7** | 33 | 14 |
| **8** | 14 | 15 |
| **9** | 23 | 21 |
| **10** | 30 | 15 |
| **11** | 22 | 14 |
| **12** | 15 | 24 |
| **13** | 30 | 22 |
| **14** | 12 | 13 |
| **15** | 16 | 17 |
| **16** | 18 | 16 |
| **17** | 14 | 14 |
| **18** | 12 | 29 |
| **average** | **19.94** | **20.00** |
| **s** | **7.34** | **6.09** |
| **min** | **12** | **13** |
| **max** | **33** | **30** |

There are no statistically significant differences between individuals when detecting the final point in the *in vitro* disintegration test (p=0,9804)

**Table III: In vivo disintegration time (seconds)**

| **Orally disintegrating placebo tablets Example 1** | | |
|---|---|---|
| **Num.** | **ANALYST 1** | **ANALYST 2** |
| **1** | 13 | 9 |
| **2** | 11 | 12 |
| **3** | 11 | 14 |
| **4** | 17 | 13 |
| **5** | 11 | 13 |
| **6** | 7 | 11 |
| **7** | 10 | 11 |
| **8** | 12 | 9 |
| **9** | 10 | 9 |
| **10** | 16 | 9 |
| **average** | **11.8** | **11.0** |
| **s** | **2.94** | **1.94** |
| **min** | **7** | **9** |
| **max** | **17** | **14** |

There are no statistically significant differences between individuals when detecting the final point in the *in vivo* disintegration test (p=0,4817). However, there are differences between the *"in vivo*" and *"in vitro"* disintegration test (p<0,05). In general, the values obtained in the *in vitro* test are higher than those obtained *in vivo.*

### EXAMPLES 2 TO 6

Five orally disintegrating placebo tablet compounds were prepared to determine the optimum content of the disintegrating system and the proposed diluent, using the general process initially described and with the compositions as detailed in Table IV. The results obtained in the characterisation of the tablets are given in Table V.

**Table IV: Orally disintegrating placebo tablets**

| **Composition for 100 g** | | | | | |
|---|---|---|---|---|---|
| **Ingredients** | **Quantity (g)** | | | | |
| | **Ex. 2** | **Ex.3** | **Ex.4** | **Ex. 5** | **Ex.6** |
| Spray-dried mannitol | 84 | 74 | 79 | - | 81 |
| Direct compression dextrose | - | - | - | 79 | - |
| Microcrystalline cellulose | 10 | 20 | 15 | 15 | 15 |
| Sodium croscarmellose | 5 | 5 | 5 | 5 | 3 |
| Magnesium stearate | 1 | 1 | 1 | 1 | 1 |

**Table V: Characterisation of the tablets in examples 2-6**

| **Parameters** | **Ex. 2** | **Ex.3** | **Ex.4** | **Ex.5** | **Ex.6** |
|---|---|---|---|---|---|
| Shape | Round 9 mm, flat, bevelled | | | | |
| Average weight (mg) | 147.5 | 146.2 | 144.5 | 151.7 | 148.5 |
| Hardness (N) | 26.2 | 25.0 | 20.7 | 23.4 | 21.9 |
| Thickness (mm) | 2.09 | 2.12 | 2.15 | 2.09 | 2.12 |
| Tensile strength (N/mm²) | 0.9 | 0.8. | 0.7 | 0.8 | 0.7 |
| Friability (%) | 0.46 | 0.07 | 0.07 | 0.84 | 0.14 |
| *In vitro* disintegration time (s) | 24 | 21 | 19 | 27 | 18 |
| *In vivo* disintegration time (s) | 20 | 12 | 11 | 18 | 13 |
| Palatability | Residue | Residue (+) | Residue | Residue (++) | Correct |

The results obtained from this series of experiments corroborate the ideal nature of the promoter system of the disintegration proposed in the present invention.

### EXAMPLE 7

A mixture of orally disintegrating tablets of ondansetron was prepared, using the general process initially described and with the composition given in Table VI. To determine the impact of the shape and dimensions of the tablet on the disintegration time, the compound was compressed with three different formats. The results obtained are given in Table VII.

**Table VI: Orally disintegrating tablets of 8 mg of ondansetron**

| **Composition for 100 g** | |
|---|---|
| **Ingredients** | **Quantity (g)** |
| Ondansetron base | 5.3 |
| Spray-dried mannitol | 73.1 |
| Microcrystalline cellulose | 15.0 |
| Sodium croscarmellose | 3 |
| Aspartame | 1.3 |
| Mint flavour | 1.3 |
| Magnesium stearate | 1.0 |

**Table VII: Characterisation of the tablets in example 7**

| **Parameters** | **Ex. 7a** | **Ex.7b** | **Ex.7c** |
|---|---|---|---|
| Shape | Round | Round | Round |
| | 8 mm | 9,0 mm | 9,0 mm |
| | Flat bevelled | Flat bevelled | biconvex |
| Average weight (mg) | 153.1 | 150.4 | 149.1 |
| | (151.4-157.8) | (147.2-153.8) | (147.4-153.2) |
| Hardness (N) | 22.3 (19-29) | 21.5 (18-27) | 23.1 (20-28) |
| Thickness (mm) | 2.75 | 2.17 | 2.32 |
| | (2.71-2.8) | (2.11-2.2) | (2.31-2.4) |
| Tensile strength (N/mm²) | 0.65 | 0.7 | 0.7 |
| Friability (%) | 0.2 % | 0.14 % | 0.18 % |
| *In vitro* disintegra tion time (s) | 34.8 (32-38) | 22.9 (19-26) | 38.2 (34-41) |
| *In vivo* disintegra tion time (s) | 20 (18-25) | 15 (14-16) | 24 (22-27) |

It is shown that the flat tablets disintegrate significantly faster than the convex ones and that the thickness also affects disintegration time.

### EXAMPLE 8

A mixture of orally disintegrating tablets of granisetron was prepared, using the general process initially described and with the composition and results given in Table VIII.

**Table VIII: Orally disintegrating tablets of 1 mg of granisetron**

| **Composition for 100 g** | |
|---|---|
| **Ingredients** | **Quantity (g)** |
| Granisetron base | 2.0 |
| Spray-dried mannitol | 75.0 |
| Microcrystalline cellulose | 15.0 |
| Sodium croscarmellose | 3.0 |
| Ammonium glycyrrhizinate | 0.5 |
| Aspartame | 2.0 |
| Orange flavour | 1.5 |
| Magnesium stearate | 1.0 |
| **Parameters** | **Values** |
| Shape | Round 5 mm, flat, bevelled |
| Average weight (mg) | 51.5 (42.4-58.1) |
| Hardness (N) | 23.5 (18-34) |
| Thickness (mm) | 2.02 (1.97-2.08) |
| Tensile strength (N/mm²) | 1.5 |
| Friability (%) | 0.08 |
| Apparent density (g/ml) | 1.2 |
| *In vitro* disintegration time (s) | 16.4 (13-21) |
| *In vivo* disintegration time (s) | 11 (10-14) |

### EXAMPLE 9

A mixture of orally disintegrating tablets of risperidone was prepared, using the general process initially described and with the composition and results given in Table IX. The results obtained in the characterisation of the tablets are also given in Table IX.

**Table IX: Orally disintegrating tablets of 1 mg of risperidone**

| **Composition for 100 g** | |
|---|---|
| **Ingredients** | **Quantity (g)** |
| Risperidone | 1.0 |
| Spray-dried mannitol | 77.5 |
| Microcrystalline cellulose | 15.0 |
| Sodium croscarmellose | 1.5 |
| Ammonium glycyrrhizinate | 0.5 |
| Aspartame | 2.0 |
| Orange flavour | 1.5 |
| Magnesium stearate | 1.0 |
| **Parameters** | **Values** |
| Shape | Round 7.5 mm, flat, bevelled |
| Average weight (mg) | 102.1 (93.2-106.1) |
| Hardness (N) | 21.5 (16-42) |
| Thickness (mm) | 2.01 (1.93-2.06) |
| Tensile strength (N/mm²) | 0.9 |
| Friability (%) | 0.2 |
| Apparent density (g/ml) | 1.17 |
| *In vitro* disintegration time (s) | 19.7 (16-24) |
| *In vivo* disintegration time (s) | 12-15 |

### EXAMPLE 10

A mixture of orally disintegrating tablets of fluoxetine was prepared, using the general process initially described and with the composition and results given in Table X. The results obtained in the characterisation of the tablets are also given in Table X.

**Table X: Orally disintegrating tablets of 20 mg of fluoxetine**

| **Composition for 100 g** | |
|---|---|
| **Ingredients** | **Quantity (g)** |
| Fluoxetine hydrochloride | 7.5 |
| Spray-dried mannitol | 71.0 |
| Microcrystalline cellulose | 15.0 |
| Sodium croscarmellose | 3.0 |
| Ammonium glycyrrhizinate | 0.3 |
| Aspartame | 1.0 |
| L-menthol | 0.2 |
| Mint flavouring | 1.0 |
| Magnesium stearate | 1.0 |
| **Parameters** | **Values** |
| Shape | Round 13 mm, flat, bevelled |
| Average weight (mg) | 301.3 (298.2-304.1) |
| Hardness (N) | 34 (29-37) |
| Thickness (mm) | 1.92 |
| Tensile strength (N/mm²) | 0.9 |
| Friability (%) | 0.31 |
| Apparent density (g/ml) | 1.18 |
| *In vitro* disintegration time (s) | 32.4 (28-36) |
| *In vivo* disintegration time (s) | 19 (16-21) |

### EXAMPLE 11

A mixture of orally disintegrating tablets of paroxetine was prepared using the general process initially described and with the composition and results given in Table XI. The results obtained in the characterisation of the tablets are also given in Table XI.

**Table XI: Orally disintegrating tablets of 20 mg of paroxetine**

| **Composition for 100 g** | |
|---|---|
| **Ingredients** | **Quantity (g)** |
| Paroxetine hydrochloride hemihydrate | 9.1 |
| Potassium polacrilin | 9.1 |
| Spray-dried mannitol | 67.6 |
| Microcrystalline cellulose | 10.0 |
| Sodium croscarmellose | 0.5 |
| Ammonium glycyrrhizinate | 0.5 |
| Aspartame | 1.0 |
| L-menthol | 0.2 |
| Mint flavouring | 1.0 |
| Magnesium stearate | 1.0 |
| **Parameters** | **Values** |
| Shape | Round 13 mm, flat, bevelled |
| Average weight (mg) | 302.1 (298.2-307.4) |
| Hardness (N) | 31 (26-34) |
| Thickness (mm) | 1.98 |
| Tensile strength (N/mm²) | 0.8 |
| Friability (%) | 0.19 |
| Apparent density (g/ml) | 1.15 |
| *In vitro* disintegration time (s) | 36.4 (33-40) |
| *In vivo* disintegration time (s) | 21 (18-24) |

## Claims

1. Tablet for oral administration that disintegrates quickly in the oral cavity in less than 30 seconds, comprising:
i) Spray-dried mannitol in a proportion of at least 59.5%;
ii) active ingredient in a proportion below or equal to 10%, as a fine powder in which at least 90% in weight of the active ingredient has a particle size less than 100 µm;
iii) Microcrystalline cellulose in a proportion from 10 to 18%, with an average particle size of approximately 50 µm where at least 99% in weight of microcrystalline cellulose has a particle size below 250 µm;.
iv) Sodium croscarmellose in a proportion from 1 to 4%; and
v) A lubricant agent in a proportion from 0.5 to 2% in weight,
where, unless specified otherwise, the percentages are expressed in weight of the total weight of the tablet.

2. Tablet for oral administration according to claim 1, **characterised in that** it has a friability below 0.5% according to Ph. Eur. 2.9.7.

3. Tablet for oral administration according to claim 2, **characterised in that** it has a friability below 0.2% according to Ph. Eur. 2.9.7.

4. Tablet for oral administration according to claim 1, **characterised in that** it has an apparent density from 1.1 to 1.3 g/ml.

5. Tablet for oral administration according to claim 1, **characterised in that** it has a flavouring agent in a proportion from 0.5 to 2% in weight of the total weight of the tablet.

6. Tablet for oral administration according to claim 5, **characterised in that** it has an artificial sweetener in a proportion from 0.5 to 2% in weight of the total weight of the tablet.

7. Tablet for oral administration according to claim 1, **characterised in that** it has a humidity adsorbing agent in a proportion from 0.1 to 0.5% in weight of the total weight of the tablet.

8. Tablet for oral administration according to claim 1, **characterised in that** it has an anti-adherent agent in a proportion from 0.5 to 2% in weight of the total weight of the tablet.

9. Tablet for oral administration according to claim 1, **characterised in that** the proportion of insoluble elements is below 20% in weight of the total weight of the tablet.

10. Tablet for oral administration according to any of previous claims, **characterised in that** it has a round shape, flat, bevelled with a thickness from 1.8 to 2.2 mm.

11. Tablet for oral administration according to claim 10, **characterised in that** it disintegrates quickly in the oral cavity in less than 20 seconds.

12. Process for obtaining a tablet for oral administration as defined in any of claims 1 to 11, **characterised in that** it comprises the following steps:
i) Sieving and mixing the components except for the lubricant agent;
ii) Sieving the lubricant agent;
iii) Mixing of all the components; and
iv) Direct compression of the final mixture.

13. Process for obtaining a tablet according to claim 12, **characterised in that** said final mixture has a flowability below or equal to 10 seconds according to Ph. Eur. 2.9.16.

14. Process for obtaining a tablet according to claim 12, **characterised in that** said final mixture has an ability to settle below or equal to 20 ml according to Ph. Eur. 2.9.15.

## Patentansprüche

1. Tablette zur oralen Verabreichung, die sich in der Mundhöhle in weniger als 30 Sekunden schnell zersetzt, umfassend:
i) Sprühgetrocknetes Mannitol in einem Anteil von zumindest 59,5%;
ii) Wirksamer Bestandteil in einem Anteil von unter oder gleich 10%, in Form eines feinen Pulvers, in welchem zumindest 90 Gew.% des aktiven Bestandteils eine Teilchengröße von unter 100 µm aufweisen;
iii) Mikrokristalline Zellulose in einem Anteil von 10 bis 18%, mit einer mittleren Teilchengröße von ungefähr 50 µm, wobei zumindest 99 Gew.% der mikrokristallinen Zellulose eine Teilchengröße von unter 250 µm aufweist;
iv) Natrium Croscarmellose in einem Anteil von 1 bis 4%; und
v) ein Gleitmittel in einem Anteil von 0,5 bis 2 Gew.%,
wobei, wenn nicht anders angegeben, die Prozentangaben als Gewicht des Gesamtgewichts der Tablette angegeben sind.

2. Tablette zur oralen Verabreichung nach Anspruch 1, **dadurch gekennzeichnet, dass** sie eine Zerfallsneigung von unter 0,5% gemäß Ph. Eur. 2.9.7. aufweist.

3. Tablette zur oralen Verabreichung nach Anspruch 2, **dadurch gekennzeichnet, dass** sie eine Zerfallsneigung von unter 0,2% gemäß Ph. Eur. 2.9.7. aufweist.

4. Tablette zur oralen Verabreichung nach Anspruch 1, **dadurch gekennzeichnet, dass** sie eine Schüttdichte von 1,1 bis 1,3 g/ml aufweist.

5. Tablette zur oralen Verabreichung nach Anspruch 1, **dadurch gekennzeichnet, dass** sie einen Geschmacksstoff in einem Anteil von 0,5 bis 2 Gew.% des Gesamtgewichts der Tablette aufweist.

6. Tablette zur oralen Verabreichung nach Anspruch 5, **dadurch gekennzeichnet, dass** sie einen künstlichen Süßstoff in einem Anteil von 0,5 bis 2 Gew.% des Gesamtgewichts der Tablette aufweist.

7. Tablette zur oralen Verabreichung nach Anspruch 1, **dadurch gekennzeichnet, dass** sie ein feuchtigkeitsadsorbierendes Mittel in einem Anteil von 0,1 bis 0,5 Gew.% des Gesamtgewichts der Tablette aufweist.

8. Tablette zur oralen Verabreichung nach Anspruch 1, **dadurch gekennzeichnet, dass** sie ein Antihaftmittel in einem Anteil von 0,5 bis 2 Gew.% des Gesamtgewichts der Tablette aufweist.

9. Tablette zur oralen Verabreichung nach Anspruch 1, **dadurch gekennzeichnet, dass** der Anteil der unlöslichen Bestandteile unter 20 Gew.% des Gesamtgewichts der Tablette ist.

10. Tablette zur oralen Verabreichung nach einem der vorhergehenden Ansprüche, **dadurch gekennzeichnet, dass** sie eine runde Form aufweist, flach ist und abgeflacht bzw. abgeschrägt ist, mit einer Dicke von 1,8 bis 2,2 mm.

11. Tablette zur oralen Verabreichung nach Anspruch 10, **dadurch gekennzeichnet, dass** sie sich in der Mundhöhle in weniger als 20 Sekunden schnell zersetzt.

12. Verfahren, um eine Tablette zur oralen Verabreichung, wie sie in einem der Ansprüche 1 bis 11 definiert ist, zu erhalten, **dadurch gekennzeichnet, dass** das Verfahren die nachfolgenden Schritte umfasst:
i) Sieben und Vermischen der Bestandteile, mit Ausnahme des Gleitmittels;
ii) Sieben des Gleitmittels;
iii) Vermischen aller Bestandteile; und
iv) direktes Verpressen der Endmischung.

13. Verfahren, um eine Tablette nach Anspruch 12 zu erhalten, **dadurch gekennzeichnet, dass** die Endmischung eine Fließfähigkeit von unter oder gleich 10 Sekunden gemäß Ph. Eur. 2.9.16. besitzt.

14. Verfahren, um eine Tablette nach Anspruch 12 zu erhalten, **dadurch gekennzeichnet, dass** die Endmischung ein Absetzvermögen von unter oder gleich 20 ml gemäß Ph. Eur. 2.9.15. besitzt.

## Revendications

1. Comprimé destiné à l'administration orale qui se désintègre rapidement dans la cavité buccale en moins de 30 secondes, comprenant :
i) du mannitol séché par atomisation dans une proportion d'au moins 59,5 % ;
ii) un ingrédient actif dans une proportion inférieure ou égale à 10 %, sous forme d'une poudre fine dans laquelle au moins 90 % en poids de l'ingrédient actif a une taille de particules inférieure à 100 µm ;
iii) de la cellulose microcristalline dans une proportion de 10 % à 18 %, avec une taille moyenne de particules d'approximativement 50 µm où au moins 99 % en poids de la cellulose microcristalline présente une taille de particules inférieure à 250 µm ;
iv) de la croscarmellose de sodium dans une proportion de 1 % à 4 % ; et
v) un agent lubrifiant dans une proportion de 0,5 % à 2 % en poids,
où, sauf indication contraire, les pourcentages sont exprimés en poids du poids total du comprimé.

2. Comprimé destiné à l'administration orale selon la revendication 1, **caractérisé en ce qu'**il présente une friabilité inférieure à 0,5 % selon Ph. Eur. 2.9.7.

3. Comprimé destiné à l'administration orale selon la revendication 2, **caractérisé en ce qu'**il présente une friabilité inférieure à 0,2 % selon Ph. Eur. 2.9.7.

4. Comprimé destiné à l'administration orale selon la revendication 1 , **caractérisé en ce qu'**il possède une densité apparente de 1,1 à 1,3 g/ml.

5. Comprimé destiné à l'administration orale selon la revendication 1, **caractérisé en ce qu'**il possède un agent aromatisant dans une proportion de 0,5 % à 2 % en poids du poids total du comprimé.

6. Comprimé destiné à l'administration orale selon la revendication 5, **caractérisé en ce qu'**il possède un édulcorant artificiel dans une proportion de 0,5 % à 2 % en poids du poids total du comprimé.

7. Comprimé destiné à l'administration orale selon la revendication 1, **caractérisé en ce qu'**il possède un agent adsorbant l'humidité dans une proportion de 0,1 % à 0,5 % en poids du poids total du comprimé.

8. Comprimé destiné à l'administration orale selon la revendication 1, **caractérisé en ce qu'**il possède un agent anti-adhérent dans une proportion de 0,5 % à 2 % en poids du poids total du comprimé.

9. Comprimé destiné à l'administration orale selon la revendication 1, **caractérisé en ce que** la proportion d'éléments insolubles est inférieure à 20 % en poids du poids total du comprimé.

10. Comprimé destiné à l'administration orale selon l'une quelconque des revendications précédentes, **caractérisé en ce qu'**il a une forme ronde, plane, biseautée avec une épaisseur de 1,8 mm à 2,2 mm.

11. Comprimé destiné à l'administration orale selon la revendication 10, **caractérisé en ce qu'**il se désintègre rapidement dans la cavité buccale en moins de 20 secondes.

12. Procédé pour obtenir un comprimé destiné à l'administration orale selon l'une quelconque des revendications 1 à 11, **caractérisé en ce qu'**il comprend les étapes suivantes :
i) tamisage et mélange des composants à l'exception de l'agent lubrifiant ;
ii) tamisage de l'agent lubrifiant ;
iii) mélange de tous les composants ; et
iv) compression directe du mélange final.

13. Procédé pour obtenir un comprimé selon la revendication 12, **caractérisé en ce que** ledit mélange final présente une fluidité inférieure ou égale à 10 secondes selon Ph. Eur. 2.9.16.

14. Procédé pour obtenir un comprimé selon la revendication 12, **caractérisé en ce que** ledit mélange final possède une capacité à se déposer inférieure ou égale à 20 ml selon Ph. Eur. 2.9.15.
